# EUROPEAN PATENT SPECIFICATION

(11) **EP 0 522 666 B1**
(45) Date of publication and mention of the grant of the patent: **23.10.1996**
(21) Application number: 92202404.7
(22) Date of filing: 22.12.1987
(51) Int. Cl.: C07C 1/06

(54) **Process for reducing methane formation and increasing liquid yields in a Fischer-Tropsch process**
Verfahren zur Verringerung der Methanbildung und zur Steigerung der Ausbeute an flüssigen Produkten in einem Fischer-Tropsch-Verfahren
Procédé pour diminuer la formation de méthane et pour augmenter le rendement en produits liquides dans un procédé de Fischer-Tropsch

(43) Date of publication of application: 13.01.1993
(62) Divisional of application: 87311323.7
(73) Proprietor: EXXON RESEARCH AND ENGINEERING COMPANY, Florham Park, New Jersey 07932-0390 (US)
(72) Inventor: Iglesia, Enrique, Clinton, New Jersey 08809 (US); Madon, Rostam Jal, Flemington, New Jersey 08822 (US)
(74) Representative: Somers, Harold Arnold

(56) References cited:
- US-A- 4 547 525

## Description

The present invention relates to a process for reducing methane production and increasing liquid yields in Fischer-Tropsch reactions.

US-A-4547525 describes and claims a process for reducing methane formation in a Fischer-Tropsch process for synthesizing hydrocarbons which comprises contacting, at an elevated temperature of 100°-500°C and a pressure of from about 100-10,000 kPa, a H₂/CO feed mixture with a heterogeneous catalyst comprising one or more Group VIII metals supported on an inorganic refractory oxide support for a time sufficient to produce hydrocarbons, including methane, wherein one or more olefins is added to the H₂/CO feed mixture in an amount sufficient to reduce said methane formation to a level lower than it would be without adding said olefin to said feed. Preferably, one or more olefins are added to said feed in an amount, based on an olefin to CO mole ratio, ranging from about 1:100 to 5:1, and preferably said olefin comprises one or more alpha olefins.

Our European patent EP-B-0321616, from which the present patent application has been divided, describes and claims a process for reducing methane formation and for increasing liquid (C₅+) yields in a Fischer-Tropsch process for synthesizing hydrocarbons from CO and H₂, comprising adding at least one olefin to a catalyst-containing reactor bed, characterized in that a gas mixture containing CO and H₂ is introduced at the inlet end of the reactor bed and the olefin is separately introduced directly into the bed at a position which is more than 10% of the length of the bed from the inlet end of the bed and more than 10% of the length of the bed from the outlet end of the bed.

The present invention provides a process for reducing methane formation and for increasing liquid (C₅₊) yields in a Fischer-Tropsch process for synthesizing hydrocarbons by introducing CO and H₂ and at least one olefin at the inlet end of a catalyst-containing reactor bed, characterized in that water vapor is used as co-feed in the process.

It has been discovered, in accordance with this invention, that liquid (C₅+) yields and methane production in Fischer-Tropsch hydrocarbon synthesis reactions are respectively increased and reduced by adding one or more olefins and water vapor into the inlet end of the reactor bed. The olefin may be obtained by separation from the product stream and recycled to the reactor bed or from an independent source, such as processing the paraffinic product of the Fischer-Tropsch reaction through a dehydrogenation reactor to convert the paraffins into olefins. In a preferred embodiment, the olefin or olefins may comprise one or more C₂-C₂₀ alpha olefins, wherein the olefin to CO mole ratio in the reactor may be in the range of from 1:100 to 5:1, e.g., from 1:20 to to 5:1, more preferably from 1:10 to 2:1, and most preferably from about 1:5 to 1:1. The molar ratio of water to olefin may be in the range from 0.1 to 50, more preferably from 0.2 to 10 and most preferably from 0.5 to 10. The catalyst may comprise at least one Group VIII metal supported on an inorganic refractory oxide support. In a particularly preferred embodiment, the catalyst will comprise ruthenium supported on titania. Cobalt or iron catalyst may also be employed.

The molar ratio of water to CO may be in the range of from 0.1:1 to 5.0:1.

Preferred olefins useful in the process of the instant invention include alpha olefins of the type R-CH=CH₂ wherein R is hydrogen or an alkyl group having 1 to 17 carbon atoms, more preferably the alkyl group has 1 to 11 carbon atoms, and most preferably the alkyl group has 1 to 8 carbon atoms. Still more preferably are C₂-C₁₀ alpha olefins. The amount of alpha olefin recycled to the reactor bed will be sufficient to maintain an olefin to CO mole ratio of from 1/100 to 5/1, more preferably 1/20 to 5/1, and most preferably 1/10 to 2/1. Also, internal olefins where unsaturation is away from the terminal carbon can also be added beneficially to the CO/H₂ feed.

Although the process of the instant invention may be practiced in the presence of any suitable Fischer-Tropsch catalyst, in a preferred embodiment it will be practiced in the presence of a catalyst comprising one or more Group VIII metals supported on an inorganic refractory oxide support, preferably ruthenium or cobalt supported on such a support. Thus, suitable supports include oxides of titania, niobia, vanadium, tantalum, silica, alumina, manganese and mixtures thereof. Preferably the catalyst support will be selected from the group consisting of titania, zirconium titanate, mixtures of titania and alumina, mixtures of titania and silica, alkaline earth titanates, alkali titanates, rare earth titanates and mixtures of any one of the foregoing with supports selected from the group consisting of vanadia, niobia, tantala, alumina, silica and mixtures thereof. Thus, in a particularly preferred embodiment of this invention the process will be carried out in the presence of a catalyst comprising ruthenium supported on a titania support. Alternatively, the catalyst can comprise cobalt or iron supported on one of the aforementioned inorganic refractory oxides.

In general, the amount of cobalt catalytic metal present is 1 to 50 weight percent of the total catalyst composition, more preferably from 10.0 to 25 weight percent.

Iron catalysts containing 10 to 60 weight percent iron, more preferably 20 to 60 weight percent, and most preferably 30 to 50 weight percent, are unsupported, but promoted with refractory metal oxide (SiO₂, Al₂O₃, etc.), alkali (K, Na, Rb) and Group IB metal (Cu, Ag). These catalysts are usually calcined, but usually not reduced, rather they are brought up to reaction temperature directly in the CO/H₂ feed.

In general, the amount of ruthenium catalytic metal present on the catalyst will generally range from 0.01 to 50 weight percent of the total catalyst composition, more preferably from 0.1 to 5.0 weight percent and most preferably from about 0.5 to about 5 weight percent.

In general, in the process of this invention the Fischer-Tropsch reactor temperature will broadly range from 100°C to 500°C, more preferably from 150°C to 300°C and most preferably 200°C to 270°C, at a pressure of 100 to 10,000 kPa, more preferably 300 to 5,000 kPa and most preferably 500 to 3,000 kPa. The space velocity (V₁) of the feed gas (H₂+CO) will range from 10 to 10,000 standard cm³/hr-(cm³ of catalyst), more preferably 100-4,000 and most preferably 200 to 2,000. The space velocity (V₂) of the alpha olefin will range from 0.1 to about 20,000 standard cm³/hr-(cm³ of catalyst), more preferably 3-4,000 and most preferably 10 to 1,000. Thus, the volume ratio of V₁/V₂ is 0. 005 to 10,000, more preferably 0.02 to 1,000 and most preferably 0.5 to 200. The hydrogen to carbon monoxide mole ratio of the feed gas, H₂/CO, will range from 0.5 to 10 and preferably from 1 to 3.

The reactor bed can comprise a single reactor bed of one catalyst system or it can comprise two catalyst beds with different Fischer-Tropsch synthesis catalysts separated by an inert inter-stage knock-out zone.

The invention will be more readily understood by reference to the following description and Examples.

### Fischer-Tropsch Hydrocarbon Synthesis

### Experimental Procedure

Anywhere from about 5 to 40 g of catalyst was loaded into a stainless steel reactor with an ID of 0.78 cm, having a thermocouple placed within the catalyst bed to measure temperature. The reactor was flushed with He at a flow rate of 500 cc/min. and then pressurized (in He) at 30 atmospheres to confirm the integrity of the unit. The He in the reactor was then brought back to 1 atmosphere and replaced with H₂ at a space velocity between 200-600 v/v/hr at room temperature. The temperature was raised in approximately 2 to 3 hours to a final reduction temperature of between 400°C to 500°C and held at this temperature overnight.

After reduction, each catalyst was brought on stream in either one of two ways, which were found to be substantially equivalent. In one method, the reduced catalyst was treated for 15 to 50 hours at 1 atmosphere 2H₂/CO at 200°C (the space velocity of the H₂/CO used was varied from 200-1,000 cm³H₂+CO h. (cc catalyst). The conditions in the reactor were then adjusted to the desired temperature, pressure and space velocity. In the other method, the atmospheric treatment in H₂/CO was not done and the catalysts were brought on-stream at the desired experimental pressure in H₂/CO, but an initial low temperature of 150-170°C, which was then slowly raised (in 1 to 5 hours) to the desired experimental temperature.

### EXAMPLES

In all of the Examples, the determination of the Fischer-Tropsch synthesis activity towards the various products was measured by employing an on-line gas chromatograph using an isothermal, fixed-bed, stainless steel reactor (0.78 cm ID) into which from about 5 to 40 g of catalyst was loaded. A thermocouple was placed within the catalyst bed to measure the reaction temperature.

### Preparation of Ru Catalyst

Titanium dioxide supports prepared by pressing Degussa P-25 powder (20-70% rutile) into wafers using a hydraulic press at a pressure of about 20,000 lb/in² (137.9MPa). The wafers were then crushed in a mortar to a coarse powder and sieved to retain the 80-140 mesh (U.S. standard) fraction for all the TiO₂ supported catalysts. The so-formed titanium dioxide support samples were calcined in flowing air overnight (about 1 liter per minute) at a temperature of 550°C to 600°C. The calcined samples were then reduced in hydrogen flowing at a rate of 1 liter per minute by heating from room temperature to 450°C for 4 hours, after which the sample was cooled in flowing H₂, flushed with He and discharged. These reduced samples possessed the characteristic blue-gray color of surface-reduced TiO₂.

Catalysts were produced by impregnating the support material with a ruthenium nitrate/acetone mixture. The nitrate was obtained from Engelhardt as 10% weight ruthenium metal in nitric acid.

The nitrate-impregnated TiO₂ was reduced in flowing hydrogen (1 1/min.) as follows: (a) heated from room temperature to 100°C and held for 0.5 hours; (b) heated from 100°C to 450°C at 3°C/min. and held for 4 hours; (c) cooled in H₂ to room temperature overnight; and (d) flushed in the reduction cell with He or Ar (1 1/min.) for one hour. Each sample of reduced catalyst was then passivated by introducing 1% O₂ into the He stream and then increasing the O₂ content to 100% over a period of from about 2-3 hours.

### Preparation of Cobalt Catalyst

Degussa P-25 TiO₂ was treated with acetone, dried at 90°C and mulled with 9.6 weight percent Sterotex (vegetable stearine). This material was tableted to 3/8 inch (9.525 mm) size, using a pilling pressure which yielded a material of 0.35-0.40 ml/g pore volume. The 3/8 inch (9.525 mm) pills were crushed and screened to 42-80 mesh size and calcined in flowing air at 500°C for 4 hours to burn out the sterotex. Portions were then re-calcined in flowing air at 650°C for 16 hours to obtain a high rutile content and screened to 80-150 mesh size. The support composite used to prepare this catalyst was 97% rutile, 13.6 m²/g, 0.173 ml/g.

### Impregnation Procedure

108.8 g cobaltous nitrate (Co(NO₃)₂ . 6H₂O and 16 ml agueous perrhenic acid solution (52 mg Re/ml) were then dissolved in about 300 ml acetone in a 1 liter round bottom flask. 160 g TiO₂ (YAX-0487) was added and the mixture placed on a rotary evaporator until dry. The catalyst was dried in a vacuum oven at 140°C for 18 hours and calcined in flowing air at 250°C for 3 hours. The calcined catalyst was re-screened to remove the small amount of fines generated during preparation.

### Cobalt Catalyst Preparation

Analytical inspections for the catalyst are given below:

| | |
|---|---|
| Cobalt, Wt. % | 11.3 |
| Rhenium, Wt. % | 0.42 |
| Sodium ppm | 369 |
| % Rutile (ASTM D 3720-78) | 97 |
| Surface Area, m₂/g | 14.6 |
| Pore Volume, ml/g | 0.122 |
| Bulk Density, g/ml | 1.41 |

Besides quantifying rutile content, the x-ray diffraction spectrum indicates the presence of CO₃O₄.

### Comparative Example 1

### Effect of Ethylene Feed-Cobalt Catalysts

The general procedure described for Fischer-Tropsch hydrocarbon synthesis was modified as follows in Examples 1 and 2.

The catalyst was started in H₂/CO at a space velocity required to achieve 25-45% CO conversion and at 1,700-2,500 Kpa. An ethylene/argon mixture was mixed with H₂/CO feed and then introduced to reactor at inlet at an amount of ethylene to give 2.5% and 6.2% ethylene in feed on Co and Ru catalysts, respectively. The total reactor pressure was then increased to the level necessary to maintain the H₂ + CO partial pressure in the reactor at the level found before ethylene addition. The reactor was operated at these conditions for 4 to 24 hours. Ethylene/argon feed was then directed so that it entered the reactor, undiluted by H₂+CO, at a point in the reactor 1/3 x L (L reactor length) down from inlet, while maintaining H₂ + CO space velocity and partial pressure at its previous level. The reactor was operated at these conditions for 4 to 24 hours. The ethylene/argon feed was discontinued and reactor conditions returned to those of beginning in order to rule out irreversible deleterious effects of ethylene addition on synthesis activity and selectivity.

| | | |
|---|---|---|
| Run | 119-342 | 119-339 |
| % Ethylene in Feed | 0 | 2.5 |
| Location Added | None | Inlet |

| Ethylene | | |
|---|---|---|
| % Ethylene Converted | - | 99.5 |

| Selectivity (%) to | | |
|---|---|---|
| C₂H₆ | - | 86.5 |
| C₃⁺ | - | 13.5 |
| C₃⁺ Yield (%) | - | 13.5 |

| Selectivity (%)* | | |
|---|---|---|
| CH₄ | 9.4 | 8.3 |
| C₂ | 1.3 | - |
| C₃ | 2.7 | 3.3 |
| C₄ | 2.4 | 2.9 |
| CO₂ | 0.4 | 0.5 |
| C₅⁺ | 83.8 | 92.9 |

| | | |
|---|---|---|
| * % of ethylene converted which appeared as these product. | | |
| ** % of ethylene added which appeared as these products. | | |
| *** Selectivity is defined as percent of the CO converted which appears as a given product (it is almost identical to weight percent). However, when ethylene is added some of the C-atoms come from it and the sum of selectivities is greater than 100%. | | |
| 11.7% Co/0.5 Re/TiO₂, 200°C, 2,050 kPa, H₂ + CO, H₂/CO = 2.1/1 25-27% CO conversion | | |

The addition of ethylene (C₂⁼) at reactor inlet lowers the CH₄ selectivity and increases C₅⁺ selectivity. Most of the ethylene (at 2.5% concentration) is converted, but only 13.5% of the converted ethylene appears as desirable C₃⁺ products; the rest (86.5%) is hydrogenated to ethane, which is unreactive towards further chain growth to C₃⁺ at Fischer-Tropsch synthesis conditions and, thus, cannot be recycled without converting it to ethylene in a dehydrogenation reactor.

### Comparative Example 2

### Effect of Ethylene Feed-Ruthenium Catalysts

| | | |
|---|---|---|
| Run | 121-455 | 121-454 |
| % Ethylene in Feed | 0 | 6.2 |
| Location Added | None | Inlet |

| Ethylene | | |
|---|---|---|
| % Ethylene Converted | - | 97 |

| Selectivity to (%)* | | |
|---|---|---|
| Ethane | - | 82 |
| C₃⁺ | - | 18 |
| Yield (%) C₃⁺** | - | 17 |

| Selectivity (%)*** | | |
|---|---|---|
| CH₄ | 5.5 | 4.3 |
| C₂ | 0.7 | - |
| C₃ | 2.1 | 2.6 |
| C₄ | 3.5 | 4.0 |
| CO₂ | 0.7 | 0.7 |
| c₅⁺ | 87.5 | 105.6 |

| | | |
|---|---|---|
| * % of ethylene converted which appeared as these products. | | |
| ** % of ethylene added which appeared as these products. | | |
| *** Selectivity is defined as percent of the CO converted which appears as a given product (it is almost identical to weight percent). However, when ethylene is added some of the C-atoms come from it and the sum of selectivities is greater than 100%. | | |
| 1.2% Ru/TiO₂, 200°C, 1,700 kPa, H₂/CO = 2.1/1, 35-45% CO conversion. | | |

Addition of ethylene to H₂/CO feed at reactor inlet (at 6.2% level) lowers the CH₄ selectivity from 5.5 to 4.3, while increasing the C₅⁺ selectivity from 87.5 to 105.6%. Most of the ethylene added in (97%) is converted, of which only 18% appears as desirable C₃⁺ products; the rest (82%) is hydrogenated to ethane, which is unreactive towards further chain growth to C₃⁺ at Fischer-Tropsch synthesis conditions and, thus, cannot be recycled without converting it to ethylene in a dehydrogenation reactor.

### Comparative Example 3

### Top versus Below Inlet Feed Ethylene-Ru

There is a clear advantage to bypassing the reactor inlet by introducing the C₂⁼ below the inlet at the same concentration. The CH₄ selectivity decreases further (from 4.3% to 3.9%), the C₅⁺ selectivity increases (from 105.6% to 117.0%) and the activity of the catalyst is unaffected by the presence of ethylene, either at inlet or below the inlet of the reactor. The apparent reason for the improved product selectivity is an increase in the yield of C₃⁺ from ethylene (the percentage of the ethylene feed converted to C₃⁺) from 17% to 26% in spite of the lower ethylene conversion when added below the inlet. Below inlet addition dramatically decreases the hydrogenation selectivity (from 82% to 44%), while increasing the C₃⁺ selectivity (from 18% to 56%). Therefore, it dramatically increases the efficiency of ethylene utilization while avoiding the formation of species such as ethane which cannot be polymerized further under Fischer-Tropsch conditions.

| | | |
|---|---|---|
| Run | 121-453 | 121-454 |
| % Ethylene in Feed | 6.2 | 6.2 |
| Location Added | Below Top Third of Reactor | Top |

| Ethylene* | | |
|---|---|---|
| % Ethylene Converted | 45.3 | 97 |

| Selectivity to (%) | | |
|---|---|---|
| Ethane | 44 | 82 |
| C₃⁺ | 56 | 18 |
| Yield (%) C₃⁺** | 26 | 17 |

| Selectivity (%)*** | | |
|---|---|---|
| CH₄ | 3.9 | 4.3 |
| C₂ | - | - |
| C₃ | 3.4 | 2.6 |
| C₄ | 5.1 | 4.0 |
| CO₂ | 0.7 | 0.7 |
| C₅⁺ | 117.0 | 105.6 |

| | | |
|---|---|---|
| * % of ethylene converted which appeared as these products. | | |
| ** % of ethylene added which appeared as these products. | | |
| *** Selectivity is defined as percent of the CO converted which appears as a given product (it is almost identical to weight percent). However, when ethylene is added some of the C-atoms come from it and the sum of selectivities is greater than 100%. | | |
| 1.2% Ru/TiO₂, 200°C, 1,700 kPa, H₂/CO = 2.1/1, 35-45% CO conversion. | | |

### Comparative Example 4

### Top versus Below Inlet Feed Ethylene-Co

There is a clear advantage to bypassing the reactor inlet by introducing the C₂⁼ below the inlet at the same concentration. The CH₄ selectivity decreases further (from 8.3% to 7.2%), the C₅⁺ selectivity increases (from 92.9% to 99.3%) and the activity of the catalyst is unaffected by the presence of ethylene, either at inlet or below the inlet of the reactor. The apparent reason is an increase in the yield of C₃⁺ from ethylene (the percentage of the ethylene feed converted to C₃⁺) (from 13.% to 27.3%), in spite of the lower ethylene conversion when added below the inlet. Below inlet addition dramatically decreases the hydrogenation selectivity (from 86.5% to 69.5%), while increasing the C₃ selectivity (from 13.5% to 30.5%). Therefore, it dramatically increases the efficiency of ethylene utilization, while avoiding the formation of species such as ethane which cannot be polymerized under Fischer-Tropsch conditions.

| | | |
|---|---|---|
| Run | 119-336 | 119-339 |
| % Ethylene in Feed | 2.5 | 2.5 |
| Location Added | Below Top Third | Inlet |

| Ethylene | | |
|---|---|---|
| % Ethylene Converted | 89.5 | 99.5 |

| Selectivity (%) to | | |
|---|---|---|
| C₂H₆ | 69.5 | 86.5 |
| C₃⁺ | 30.5 | 13.5 |
| C₃⁺ Yield (%) | 27.3 | 13.5 |

| Selectivity (%) * | | |
|---|---|---|
| CH₄ | 7.2 | 8.3 |
| C₂ | - | - |
| C₃ | 3.7 | 3.3 |
| C₄ | 3.7 | 2.9 |
| CO₂ | 0.4 | 0.5 |
| C₅⁺ | 99.3 | 92.9 |

| | | |
|---|---|---|
| * % of ethylene converted which appeared as these products. | | |
| ** % of ethylene added which appeared as these products. | | |
| *** Selectivity is defined as percent of the CO converted which appears as a given product (it is almost identical to weight percent). However, when ethylene is added some of the C-atoms come from it and the sum of selectivities is greater than 100%. | | |
| 11.7% Co/0.5 Re/TiO₂, 200°C, 2,050 kPa, H₂ + Co, H₂/CO - 2.1/1 25-27% CO conversion. | | |

### Comparative Example 5

### Experimental Procedure for Ethylene Addition

The catalyst was started in H₂/CO at a space velocity required to achieve 15-30% CO conversion at 2,000-2,100 kPa. Conditions were maintained for at least 48 hours. An ethylene/argon mixture was then mixed with the CO/H₂ feed and introduced to the reactor at the inlet at an ethylene concentration of 2-7% by volume. The total reactor pressure was then increased so as to maintain H₂+CO partial pressure equal to that prior to ethylene cofeed. The reactor was operated at these conditions for at least 24 hours.

### Effect of Ethylene Addition - Ruthenium Catalyst

| | | |
|---|---|---|
| Run | 123-608 | 123-609 |
| % Ethylene in Feed | 0 | 7 |
| % Ethylene Converted | - | 73.4 |
| Selectivity to (%)* | - | |
| Ethane | - | 50.5 |
| C₃⁺ | - | 49.5 |
| Yield to (%)** | | |
| C₃⁺ | - | 36.3 |

| Rate of Ethylene Conversion (mol C₂⁼/g. atom Ru.h) | | |
|---|---|---|
| C₃⁺ | - | 6.1 |
| C₂H₆ | - | 6.3 |
| Rate of CO conversion (mol CO/g.atom Ru.H) | 12.8 | 12.5 |

| CO selectivity (%)*** | | |
|---|---|---|
| CH₄ | 3.7 | 3.0 |
| C₂ | 0.4 | - |
| C₃ | 1.75 | 2.26 |
| C₄ | 2.89 | 3.4 |
| CO₂ | 0.3 | 0.4 |
| C₅⁺ | 91.0 | 188.5 |
| 1.2% Ru/TiO₂, 191-192°C, 2,100 kPa, H₂+CO, H₂/CO = 2.1/1, 18-22% CO conversion. | | |

| | | |
|---|---|---|
| * % of ethylene converted which appeared as these products. | | |
| ** % of ethylene added which appeared as these products. | | |
| ** Selectivity is defined here as the percent of CO converted which appears as a given product. However, when ethylene is added some of the C-atoms come from it and the value of this "selectivities" can exceed 100%. | | |

The addition of ethylene (C₂⁼) to the H₂/CO feed at reactor inlet lowers the CH₄ selectivity and increases the C₅⁺ selectivity on the Ru/TiO₂ catalyst. A large fraction of the ethylene (7% concentration to the feed) added is converted (73.4%), but only 49.5% of the converted C₂⁼ appears as desired C₃⁺ products; the rest (50.5%) is hydrogenated to ethane, which is unreactive towards further chain growth at Fischer-Tropsch synthesis conditions and, thus, cannot be recycled without converting it to ethylene in a dehydrogenation reactor.

### Example

### Experimental Procedure for Ethylene Addition and Water Co-feed

The catalyst was started in H₂/CO at a space velocity required to achieve 15-30% CO conversion at 2,000-2,100 kPa. Conditions were maintained for at least 48 hours. An ethylene/argon mixture was then mixed with the CO/H₂ feed and introduced to the reactor at the inlet at an ethylene concentration of 2-7% by volume. The total reactor pressure was then increased so as to maintain H₂+CO partial pressure equal to that prior to ethylene co-feed. The reactor was operated at these conditions for at least 24 hours. Then water vapor was introduced at a concentration of 10-20 volume percent by bubbling the H₂+CO feed through a saturator maintained at 120-145°C, while keeping the ethylene/argon flow constant. The total reactor pressure was increased so as to maintain the H₂+CO and the ethylene partial pressures at their level prior to water cofeed. Also, the flow rates of H₂/CO and of ethylene/argon were increased so as to maintain the CO conversion at their level prior to water addition. The reactor was operated at these conditions for at least 24 hours.

### Effect of Ethylene/Water Addition - Ruthenium Catalyst

| | | |
|---|---|---|
| Run | 123-609 | 123-614 |
| % Ethylene in Feed | 7 | 7 |
| % Water in Feed | 0 | 18 |
| % Ethylene Converted | 73.4 | 38.2 |

| Selectivity to (%) * | | |
|---|---|---|
| Ethane | 50.5 | 11.5 |
| C₃⁺ | 49.5 | 88.5 |

| Yield (%)** | | |
|---|---|---|
| C₃⁺ | 36.3 | 33.8 |

| Rate of Ethylene Conversion to (mole C₂⁼/g.atom Ru.h) | | |
|---|---|---|
| C₃⁺ | 6.1 | 17.3 |
| C₂H₆ | 6.3 | 2.2 |
| Rate of CO Conversion (mol CO/g.atom Ru.h) | 12.5 | 34.0 |

| CO Selectivity (%)*** | | |
|---|---|---|
| CH₄ | 3.0 | 0.9 |
| C₂ | - | - |
| C₃ | 2.26 | 0.97 |
| C₄ | 3.40 | 1.46 |
| CO₂ | 0.4 | 0.4 |
| C₅⁺ | 188.5 | 198.5 |
| 1.2% Ru/TiO₂, 191-192°C, 2,100 kPa, H₂+CO, H₂/CO = 2.1/1, 18-22% CO conversion. | | |

| | | |
|---|---|---|
| * % of ethylene converted which appeared as these products. | | |
| ** % of ethylene added which appeared as these products. | | |
| *** Selectivity is defined here as the percent of CO converted which appears as a given product. However, when ethylene is added some of the C-atoms come from it and the value of this "selectivities" can exceed 100%. | | |

The Table shows that there is a clear advantage to the cofeeding of water vapor with the ethylene and with the H₂/CO feed. While the ethylene conversion decreases from 73.5% to 38.2% with the addition of 18% H₂O, the yield decreases only slightly (36.3% to 33.8%). However, most importantly:
o the selectivity to desired C₃⁺ products increases from 49.5% to 88.5%;
o the rate of ethylene conversion to C₃⁺ products and of CO conversion increase approximately threefold; in other words, the activity of the catalyst for both reactions, C₂H₄ incorporation and Fischer-Tropsch synthesis, increases threefold.
o the rate of ethylene conversion to ethane (i.e., hydrogenation rate), actually decreases approximately threefold with the addition of 18% water vapor to the H₂/CO/C₂⁼ feed.

Accompanying beneficial effects are a decrease in the CH₄ selectivity (3.0% to 0.9%) and an increase in the effective C₅⁺ selectivity from 138.5% to 199.5%.

These results suggest that the effect of water is to enhance the reincorporation and chain growth of added ethylene while dramatically inhibiting its hydrogenation to undesired ethane product.

### Notes

o Mass expressed in pounds (lbs) is converted to kg equivalent by multiplying by 0.4536.
o Length expressed in inch is converted to cm by multiplying by 2.54.

## Claims

1. A process for reducing methane formation and for increasing liquid (C₅₊) yields in a Fischer-Tropsch process for synthesizing hydrocarbons by introducing CO and H₂ and at least one olefin at the inlet end of a catalyst-containing reactor bed, characterized in that water vapor is used as co-feed in the process.

2. The process of claim 1 wherein the amount of said olefin added to said reactor bed is based on an olefin to CO mole ratio in the range of from 1:100 to 5:1.

3. The process of claim 1 or claim 2 wherein said olefin(s) is or are alpha olefin of the type R-CH=CH₂, wherein R is hydrogen or an alkyl group having 1 to 17 carbon atoms.

4. The process of any one of claims 1 to 3 wherein said olefin comprises a C₂-C₂₀ alpha olefin.

5. The process of any one of claims 1 to 4 wherein said catalyst comprises comprises one or more Group VIII catalytic metals.

6. The process of claim 5 wherein said catalytic metal is selected from iron, cobalt and ruthenium.

7. The process of any one of claims 1 to 6 performed at a temperature of at least 100°C.

8. The process of any one of claims 1 to 7 wherein the molar ratio of water to CO is in the range of from 0.1:1 to 5.0:1.

9. The process of any one of claims 1 to 8 wherein the mol ratio of water to olefin is in the range of from 0.1:1 to 50:1.

10. The process of claim 9 wherein the mol ratio of water to olefin is in the range of from 0.2:1 to 10:1.

## Patentansprüche

1. Verfahren zur Verringerung der Methanbildung und zur Erhöhung der Flüssigkeitsausbeuten (C₅₊) in einem Fischer-Tropsch-Verfahren zum Synthetisieren Von Kohlenwasserstoffen durch Einbringen von CO und H₂ und mindestens einem Olefin in den Zuführungsbereich eines Katalysator enthaltenden Reaktorbetts, dadurch gekennzeichnet, daß Wasserdampf als ebenfalls eingebrachtes Einsatzmaterial (Coeinsatzmaterial) in dem Verfahren verwendet wird.

2. Verfahren nach Anspruch 1, bei dem die Menge an dem Reaktorbett zugeführten Olefin auf einem Molverhältnis von Olefin zu CO im Bereich von 1:100 bis 5:1 basiert.

3. Verfahren nach Anspruch 1 oder Anspruch 2, bei dem das Olefin bzw. die Olefine α-Olefin des Typs R-CH=CH₂ ist bzw. sind, wobei R Wasserstoff oder eine Alkylgruppe mit 1 bis 17 Kohlenstoffatomen ist.

4. Verfahren nach einem der Ansprüche 1 bis 3, bei dem das Olefin ein C₂- bis C₂₀-α-Olefin umfaßt.

5. Verfahren nach einem der Ansprüche 1 bis 4, bei dem der Katalysator ein oder mehrere katalytische Gruppe VIII Metalle umfaßt.

6. Verfahren nach Anspruch 5, bei dem das katalytische Metall ausgewählt ist aus Eisen, Kobalt und Ruthenium.

7. Verfahren nach einem der Ansprüche 1 bis 6, das bei einer Temperatur von mindestens 100°C durchgeführt wird.

8. Verfahren nach einem der Ansprüche 1 bis 7, bei dem das Molverhältnis von Wasser zu CO im Bereich von 0,1:1 bis 5,0:1 liegt.

9. Verfahren nach einem der Ansprüche 1 bis 8, bei dem das Molverhältnis von Wasser zu Olefin im Bereich von 0,1:1 bis 50:1 liegt.

10. Verfahren nach Anspruch 9, bei dem das Molverhältnis von Wasser zu Olefin im Bereich von 0,2:1 bis 10:1 liegt.

## Revendications

1. Procédé pour réduire la formation de méthane et pour augmenter les rendements en liquides (C₅⁺) dans un procédé Fischer-Tropsch de synthèse d'hydrocarbures, par introduction de CO et de H₂ et d'au moins une oléfine à l'extrémité d'entrée d'un lit de réacteur contenant un catalyseur, caractérisé en ce qu'on utilise de la vapeur d'eau comme complément de charge dans le procédé.

2. Procédé selon la revendication 1, dans lequel la quantité de ladite oléfine ajoutée audit lit de réacteur se fonde sur un rapport en moles de l'oléfine au CO compris dans l'intervalle de 1:100 à 5:1.

3. Procédé selon la revendication 1 ou 2, dans lequel ladite oléfine ou lesdites oléfines est ou sont des α-oléfines du type R-CH=CH₂, où R est un hydrogène ou un groupe alkyle ayant de 1 à 17 atomes de carbone.

4. Procédé selon l'une quelconque des revendications 1 à 3, dans lequel ladite oléfine comprend une α-oléfine en C₂-C₂₀.

5. Procédé selon l'une quelconque des revendications 1 à 4, dans lequel ledit catalyseur comprend un ou plusieurs métaux catalytiques du Groupe VIII.

6. Procédé selon la revendication 5, dans lequel ledit métal catalytique est choisi parmi le fer, le cobalt et le ruthénium.

7. Procédé selon l'une quelconque des revendications 1 à 6, mis en oeuvre à une température d'au moins 100°C.

8. Procédé selon l'une quelconque des revendications 1 à 7, dans lequel le rapport en moles de l'eau au CO est compris dans l'intervalle de 0,1:1 à 5,0:1.

9. Procédé selon l'une quelconque des revendications 1 à 8, dans lequel le rapport en moles de l'eau à l'oléfine est compris dans l'intervalle de 0,1:1 à 50:1.

10. Procédé selon la revendication 9, dans lequel le rapport en moles de l'eau à l'oléfine est compris dans l'intervalle de 0,2:1 à 10:1.
